# EUROPEAN PATENT SPECIFICATION

(11) **EP 3 261 686 B1**
(45) Date of publication and mention of the grant of the patent: **10.05.2023**
(21) Application number: 16718501.6
(22) Date of filing: 29.02.2016
(51) Int. Cl.: A61L 27/54, A61L 27/56, A61L 27/58

(54) **DRUG ELUTING FOAMS AND THE PRODUCTION THEREOF**
MEDIKAMENTENBESCHICHTETE SCHAUMSTOFFE UND HERSTELLUNG DAVON
MOUSSES ÉLUANT DES MÉDICAMENTS ET LEUR PRODUCTION

(30) Priority: 27.02.2015 NL 2014371
(43) Date of publication of application: 03.01.2018
(60) Divisional application: 22202537.1
(73) Proprietor: Polyganics IP B.V., 9727 DL Groningen (NL)
(72) Inventor: TOOREN, Martin Franke, 9727 DL Groningen (NL); RIBBELS, Romke Stephan Rudolf, 9727 DL Groningen (NL); DENISOV, Konstantin Igorovitch, 9727 DL Groningen (NL)
(74) Representative: V.O.
(86) International application number: PCT/NL2016/050142
(87) International publication number: WO 2016/137327

(56) References cited:
- WO-A1-2004/062704
- WO-A1-2014/064140
- WO-A2-2004/041118

## Description

The invention is directed to biodegradable foams for medical applications. In particular, the invention is directed to multilayered drug eluting biodegradable foams that may be applied to tissues and/or cavities of the human or animal body.

Biodegradable foams are used in a variety of medical applications. They may be applied for packing, drainage, or for maintaining, opening or dilating bodily structures such as veins, arteries, uterus, urethras hollow body organs, nasal passages, sinus cavities, and the like.

It is advantageous if the biodegradable foams, in addition to be suitable for the above-mentioned applications, comprises one or more pharmaceutically active components, *viz.* one or more drugs. Preferably, this drug is eluted from the foam over time resulting in a controlled local release of the drug.

Beckman et al. (Acta Biomaterialia 5 (2009) 2389-2408) describes drug eluting biodegradable foams comprising drugs in the backbone of the polymers from which the foams are made. Upon degradation of the polymer, the drugs are released by the hydrolytic cleavage of the drug from the backbone of the material. This results in a controlled release of the drug. The drawback of this method however, is that only drugs that have a plurality of functional groups (v*iz.* hydroxyls) can be used. These functional groups are required to incorporate the drug into the backbone of the polymer. The number of functional groups and the reactivity are other parameters that determine which drugs can be incorporated and what type of foams may be formed. Moreover, the drugs need to have the appropriate stability so that they are not degraded during the polymerization reaction or during the degradation of the polymer in the body. Hence, the necessity of incorporating drug in the backbone of the polymer greatly limits the application of this method.

US5686091 describes a drug eluting biodegradable foam for use as a scaffold for cell transplantation. The foam comprises poly(lactic acid) and incorporates additives such as nutrients and drugs. The rate of drug release is controlled by the amount of additives that are loaded into the foam. The total amount of drugs that is released from the foam is equal to the total amount the foam was loaded with at the production. Hence, the total amount loaded drugs controls the release rate as well as the total amount of drugs that will be released from the foam. A drawback of this method is that it is not possible to independently control the rate of release and the total amount of released drugs.

WO2004/041118 describes a single foam layer that is suitable for delivering therapeutic agents.

A further drawback of the above-mentioned methods, is that it is not possible to have multiple drugs that are each released at a different rate and or in a different amount. Such a method would in fact be desirable as various medical treatments rely on multiple drugs of which the dosage regimes differ. Moreover, it is desirable to be able to control the delay with which the release of one or more drugs commences.

WO 2004/062704 A1 discloses drug eluting biodegradable foams comprising a polymer layer that is a phase-separated polymer comprising at least one amorphous segment and at least one crystalline segment and wherein said amorphous segment comprises a hydrophilic segment.

The present inventors have surprisingly found a drug eluting biodegradable foam that overcomes at least part of the above mentioned drawbacks. This is achieved by providing a multilayered drug eluting biodegradable foam comprising at least two foam layers, wherein each layer independently comprises a polymer that is a phase-separated polymer comprising at least one amorphous segment and at least one crystalline segment, and wherein said amorphous segment optionally comprises a hydrophilic segment and wherein at least one layer of said layers is a drug-comprising layer comprising at least one drug that is a mixture with the polymer present in the drug-comprising layer.

The two layers which are comprised by the multilayered drug eluting biodegradable foam of the present invention are each a foam layer. As such, the at least two layers are at least two foam layers (in other words, the term "layer" refers to "foam layer"). The drug-comprising layer is thus a drug-comprising foam layer.

The drug and the polymer being a mixture means that the drug is non-covalently incorporated into the layer. It is thus not covalently bound to the polymers by *e.g.* incorporation into the backbone of the polymer.

Foams typically comprise voids and walls. The walls comprise polymer material. The drugs and the polymer being a mixture in accordance with the present invention further means that the drug is substantially located in the wall of the foam. In the situation where a foam is soaked into a solution comprising a drug after the foam has been formed, the drug is typically substantially located in the voids of the foam and only to a minimal extent located in the walls. Without wishing to be bound by theory, it is believed that when drugs are substantially located in the voids, drug release is non-controlled and about instantaneous.
Figure 1 illustrates the tuneable period of constant drug release by varying the porosity of the layer. The illustration depicts the period of constant drug release of three different drug-comprising layers (formulation 1-3).
Figure 2 depicts the in vitro drug release profiles at different time intervals of three different drug-comprising layers (Formulation 1-3).
Figure 3 is a two-layered drug eluting biodegradable foam in accordance with the present invention.
Figure 4 is a three-layer drug eluting biodegradable foam in accordance with the present invention.
Figure 5 is a five-layered drug eluting biodegradable foam in accordance with the present invention.
Figures 6 and 7 are embodiments of the devices in accordance with the present invention.

### Multilayered foam

The terms drug elution and drug release are used herein with the same meaning.

The drug-comprising layer comprised in the multilayered foam in accordance with the present invention is obtainable by
- providing the polymer;
- dissolving the polymer in a solvent resulting in a polymer solution;
- adding the drug to the polymer solution;
- mixing the drug and polymer resulting in a drug-containing polymer solution;
- substantially removing the solvent, preferably by freeze-drying, from the drug-containing polymer solution to obtain the drug-comprising-layer.

Typical solvents that are used are cyclic ethers such as 1,4-dioxane or tetrahydrofuran (THF), aliphatic solvents such as hexane, heptane or cyclohexane, acetonitrile and mixtures thereof. With substantially removing the solvent is meant that the majority of solvent is removed such that a layer is obtained. Typically more than 90%, preferably more than 95% of the solvent is removed. Typically used solvents may be harmful or toxic and are therefore preferably removed as well as possible. Most preferably more than 99% of the solvent is removed.

By selecting the concentration of polymer in the polymer solution, the porosity of the resulting layer may be influenced. The porosity of the layers produced is typically about 85-99%, preferably 92-98%, more preferably 95-98%.

Surprisingly, the inventors have found that the porosity of the layer influences the release rate of the drug from the layer. The higher the porosity, the higher the rate of release and vice versa. Without wishing to be bound by theory, it is believed that an increased porosity results in an increased degradation rate and thereby an increase released rate.

The rate of release of the drug from the drug-releasing layer or drug eluting device (*vide infra*) may be expressed as the time required to release a certain amount of drug in a certain amount of time. For the present invention, generally 8 hours to 1.5 day are required to release 50% of the drug. In particular embodiments, it may be preferred that 50% of the drug is released in a longer time, e.g. in 1 to 5 days. To release about 100% (e.g. more than 95%) of the drug, a time of 4 to 14 days is generally preferred.

The layers in the multilayered drug eluting biodegradable foam can be distinguished by at least one chemical and/or physical property. Examples of these chemical and physical properties are density, porosity, hydrophilicity of the layer, the polymer in the layer and/or which type of drugs or other chemical additives are present in the layer. The other additives may for instance be used to visualize the degradation of the layer.

The layers may be graded layers. This means that one or more chemical and/or physical properties of the layer gradually changes over at least one dimension of the layer. It may for instance be possible that the concentration of drug changes over the thickness of the layer such that e.g. the external part of the foam (*i.e.* the site that is exposed to the body wherein the foam is placed) has a higher concentration of drug than the internal part of the foam. This may result in a gradient drug release profile.

It is preferred that the multilayered foam comprises two or more layers of drug-comprising layers which only differ in the type of drug they comprise. In a preferred embodiment of the present invention, at least two layers are drug-comprising foam layers which differ in one or more of the following properties:
- density;
- porosity;
- type of polymer;
- types of drugs; and
- hydrophilicity.

In this particular embodiment, the different layers may show different rates of drug release. The drug-comprising layers may comprise one or more drugs depending on what is preferred for the specific treatment in which the multilayered foam is used. By using different layers with different rates of drug release, a very specific drug release profile (*e.g.* a high initial release rate followed by a lower rate followed by a high final release rate) may be achieved that can not be achieved with a single drug-releasing layer. It may also be possible to influence the drug rate of a certain layer by stacking other layers on top of it, either on just one side of the layer or on both sides (*e.g.* by sandwiching the layer). The rate of biodegradation of the layer can be influenced by the adjacent stacked layers.

In a particular embodiment of the present invention, at least two layers are drug-comprising layers having different hydrophilic properties. These hydrophilic properties may also influence the rate of biodegradation and thereby the rate of release. The hydrophilic properties of the layers can be influenced by the type of polymers that are used. As described herein below in more detail, the polymers may comprise hydrophilic segments. By increasing the number and/or length of the hydrophilic segments, the hydrophilicity of the resulting layer also increases.

The hydrophilicity of the layer may also be influence by loading the layer with a hydrophilic substance as additive. Examples of such substances may be poly(ethyleneglycol) or (hygroscopic) salts. The hydrophilic substance may act similarly as a hydrophilic segment and *e.g.* influence the rate of degradation of the layer.

In a preferred embodiment of the present invention, the multilayered foam comprises one type of polymer. This means that a single type of polymer can be used for the production of the multilayered foam.

This greatly facilitates the production of the multilayered foam. It will be appreciated that having one type of polymer does not mean that the different layers are the same in terms of other chemical or physical parameters such as porosity, density and/or hydophilicity by *e.g.* added hydrophilic substances. It merely means that the polymers are structurally identical.

A particular advantage of the present invention is that no additives such as release modifiers may be required to influence the rate of drug release.

### Polymers

The multilayered drug eluting biodegradable foam in accordance with the present invention is typically non-toxic and physiologically compatible. In a preferred embodiment, the polymer is therefore selected from the group consisting of polyesters, polyethers, polyhydroxyacids, polylactones, polyetheresters, polycarbonates, polydioxanes, polyanhydrides, polyurethanes, polyester(ether)urethanes, polyurethane urea, polyamides, polyesteramides, poly-orthoesters, polyaminoacids, polyphosphonates, polyphosphazenes and combinations thereof.

Each of the at least two foam layers comprise a polymer that is a phase-separated polymer comprising at least one amorphous segment and at least one crystalline segment. Such polymers are described e.g. in WO-A-99/64491. A phase-separated polymer typically results in a layer that has good mechanical properties for medical application.
Each of the at least two foam layers in the multilayered foam comprises individually a polymer that is a phase-separated polymer comprising at least one amorphous segment and at least one crystalline segment. These foam layers are preferably directly stacked on top of each other. It was found that, in case the layers of foam comprising the phase-separated polymer are directly stacked on top of each other such that (at least part of) the surface of one layers is in direct contact with (at least part of) the surface of the adjacent layer, the layers of foam bind particularly well to each other. Without wishing to be bound by theory, it is believed the crystalline (hard) segment (as described in WO-A-2004/062704) of the polymer in the layer may form hydrogen bonds with the crystalline (hard) segment of the polymer in the adjacent layer.

In a further preferred embodiment, at least one layer is also absorbent. WO-A-2004/062704, describes polymers to create absorbent foams that are particularly preferred in the present invention. The absorbent foams of WO-A-2004/062704 comprising a phase-separated polymer comprising at least one amorphous segment and at least one crystalline segment, wherein at least said amorphous segment comprises a hydrophilic segment. The presence of the hydrophilic segment increases the absorption capacities of the layer and may also influence the rate of biodegradation.

The term "biodegradable" as used herein, refers to the ability of a polymer to be acted upon biochemically in general by living cells or organisms or part of these systems, including hydrolysis, and to degrade and disintegrate into chemical or biochemical products.

The term "bioresorbable" as used herein, refers to the ability of being completely metabolized by the human or animal body.

The term "phase-separated polymer" as used herein, refers to a polymer comprising soft (amorphous) segments, as well as hard (crystalline) segments, the hard segment having a phase transition temperature of at least mammalian body temperatures (which is generally 37 °C for humans) and the phase-separated morphology being manifest when the foam prepared from such a polymer is applied in the human or animal body for a sufficient period of time. Also, the polymer placed under temperature conditions comparable to the human or animal body exhibits said phase-separated morphology. A phase separated polymer is characterised by the presence of at least two immiscible or partly miscible phases with a different morphology at normal environmental conditions. Within one material a rubber phase and a crystalline phase (at a temperature above the glass transition temperature of the amorphous phase and below the melting temperature of the crystalline phase) may be present or a glassy and a crystalline phase (at a temperature below the glass transition temperature of the amorphous phase). Also at least two amorphous phases can be present at a temperature between the two phase transitions e.g. one glassy and one rubbery phase. At a temperature above the highest phase transition which is either a melting or glass transition temperature, the liquid and rubbery or the two rubbery phases, respectively, can form a phase mixed morphology or they can still be immiscible. Immiscible liquid and/or rubbery phases usually results in a polymer with a phase separated morphology without the initial desired mechanical properties at normal environmental conditions.

The term "amorphous" as used herein, refers to segments present in the polymer of the invention with at least one glass transition temperature below the temperature of the cavities of the human or animal body into which the foam is packed, and may also refer to a combination of an amorphous and crystalline segment which is completely amorphous when packed in the human or animal body. For example, PEG in a pre-polymer may be crystalline in pure form, but may be amorphous when comprised in the R segment of a polyurethane of the formula (I). Longer PEG segments may also be partly crystalline when comprised in the R segment of a polyurethane of the formula (I), but will become amorphous ("dissolves") when placed in contact with water. Therefore such longer PEG segments are part of the soft segment of the phase separated polymer of the formulas (I), whereas the hard segment should remain crystalline in nature to provide sufficient support to a layer in the wet and packed state for, at least, a certain period of time.

The term "crystalline" as used herein, refers to segments, present in the polymer of the invention, that are crystalline when packed in the human or animal body, *i.e.,* that have a melting temperature above the temperature of the cavities of the human or animal body into which the layer is packed.

A "hydrophilic segment" as used herein, refers to a segment comprising at least one, preferably at least two, more preferably at least three hydrophilic groups such as can be provided for instance by C-O-C, or ether, linkages. A hydrophilic segment may thus be provided by a polyether segment. A hydrophilic segment may also be provided by polypeptide, poly(vinyl alcohol), poly(vinylpyrrolidone) or poly(hydroxymethylmethacrylate). A hydrophilic segment is preferably derived from polyalkyleneglycol, such as polyethyleneglycol, polypropyleneglycol, or polybutyleneglycol. The preferred hydrophilic segment is a polyethyleneglycol (PEG) segment.

The term "segment" as used herein, refers to a polymeric structure of any length. In the art of polymer technology a long polymeric structure is often referred to as a block, whereas a short polymeric structure is often referred to as a segment. Both these conventional meanings are understood to be comprised in the term "segment" as used herein.

In one particular embodiment of the present application the polymer is of the formula:

-[R-Q¹[-R'-Z¹-[R"-Z²-R'-Z³]ₚ-R"-Z⁴]_{q}-R'-Q²]ₙ- (I)

wherein R is selected from one or more aliphatic polyesters, polyetheresters, polyethers, polyanhydrides and/or polycarbonates, and optionally at least one R comprises a hydrophilic segment, R' and R" are independently C₂-C₈ alkylene, optionally substituted with C₁-C₁₀ alkyl or C₁-C₁₀ alkyl groups substituted with halides or protected S, N, P or O moieties and/or comprising S, N, P or O in the alkylene chain, Z¹-Z⁴ are independently amide, urea or urethane, Q¹ and Q² are independently urea, urethane, amide, carbonate, ester or anhydride, n is an integer from 5-500, p and q are independent 0 or 1, provided that when q is 0, R is at least one amorphous aliphatic polyester, polyether, polyanhydride and/or polycarbonate segment with optionally at least one crystalline polyether, polyester, polyetherester or polyanhydride segment.

The simplest form of the polymer, as represented by formula I, from which a layer of the invention may be comprised is of the formula: -R-Q¹-R'-Q²-, *i.e.* when q=0.

According to the present invention, the amorphous segment is comprised in the -R- part of the polymer according to formula (I). In case q=1, the Q¹[-R'-Z¹-[R"-Z²-R'-Z³]ₚ-R"-Z⁴]_{q}-R'-Q² part of the polymer according to formula (I) represents the crystalline segment. In this particular case the amorphous and crystalline segments are alternating, thus providing the hard segment with a uniform block-length.

As described above, R may represent a mixture of two or more different types of aliphatic polyesters, polyetheresters, polyethers, polyanhydrides and/or polycarbonates, which mixture comprises both amorphous and crystalline types, so that both are comprised in a layer of the invention. In the case that a mixture of amorphous and crystalline types of R segments are provided in a polymer according to the formula (I), optionally at least one hydrophilic segment is provided in at least one amorphous R segment.

R may in particular be derived from the cyclic monomers lactide (L, D or LD), glycolide, ε-caprolactone, δ-valerolactone, trimethylenecarbonate, tetramethylenecarbonate, 1,5-dioxepane-2-one, *para*-dioxanone, and combinations thereof and optionally polyethyleneglycol, polypropyleneglycol, polybutyleneglycol and combinations thereof. In a further preferred embodiment, R is an amorphous polyester derived from exclusively lactide and ε-caprolactone, with a molecular weight between 1000 and 4000. In an even further preferred embodiment, R is about 25 wt.% lactide, about 25 wt.% ε-caprolactone and about 50 wt.% of polyethyleneglycol.

In a polymer according to the formula (I), Q¹ and Q² may be selected from amide, urea, urethane ester, carbonate or anhydride groups, whereas Z¹ through Z⁴ should be chosen from amide, urea or urethane groups so that at least 4 hydrogen bond forming groups are present in a row in the crystalline segment. The group R' in -Z²-R'-Z³- may be different or similar to R' in -Q¹-R'-Z¹- or -Z⁴-R'-Q²-.

As stated, R optionally comprises a hydrophilic segment and such a hydrophilic segment can very suitably be an ether segment, such as a polyether segment derivable from such polyether compounds as polyethyleneglycol, polypropyleneglycol or polybutyleneglycol. Also, a hydrophilic segment comprised in R may be derived from polypeptide, poly(vinyl alcohol), poly(vinylpyrrolidone) or poly(hydroxymethylmethacrylate). A hydrophilic segment is preferably a polyether such as poly(ethylene glycol), polypropylene glycol) or poly(butylene)glycol..

In case the amorphous segment comprises a hydrophilic segment, said amorphous segment preferably comprises polyethyleneglycol in a content of 1-80 wt%, more preferably 5-60 wt%, even more preferably 20-50 wt%, most preferably 50 wt%.

In a preferred embodiment, the phase-separated polymer is a polymer according to formula I, wherein R' is (CH₂)₄, R" is (CH₂)₄, or both R' and R" are (CH₂)₄. Preferably, Z¹-Z⁴ are urethane.

### Drugs

The drug-comprising layer comprises one or more drugs. In the embodiments where the multi-layered foam comprises more than one drug-comprising layer, each drug-comprising layer may individually comprise different types and concentrations of drugs.

The one or more drug that is comprised in the drug-comprising layer is in accordance with the present invention and can be any pharmaceutically active compound. It may be molecularly small compounds or larger compounds such as polysaccharides (e.g. chitosan, glycoproteins, amylopectins, polycarbonates, hyaluronic acids, celluloses and the like).

Typically, the drug is an anti-inflammatory agent, a hemostatic agent, an anti-allergen, an anti-cholinergic agent, an antihistamine, an anti-infective, an anti-platelet, an anti-coagulant, an anti-thrombic agent, an anti-scarring agent, an anti-proliferative agent, a chemotherapeutic agent, an anti-neoplastic agent, a pro-healing agent, decongestant, a vitamin, a hyperosmolar agent, an immunomodulator, an immunosuppressive agent, or combinations thereof. In a preferred embodiment, the drug is a steroidal anti-inflammatory agent. It has been found that the relatively slow release of the drug from the drug eluting multilayered foam in accordance with the present invention is particularly suitable for steroidal anti-inflammatory agents.

### Device and use thereof

The drug eluting multilayered foam in accordance with the present invention may be used to partially cover or coat the surface of a medical device such as a tube, stent, sheet, mesh, wire or another type of foam. The drugs eluting foam or the medical device in accordance with the present invention may typically be used for temporary wound dressing, control of bleeding, absorption of fluids, tissue support, packing, drainage of fluids, or for maintaining, opening or dilating bodily structures, providing pressure to tissues or combinations thereof. Since the foam of the invention comprises one or more drugs, a further use of the device may be the local delivery of drugs to tissues and/or cavities

The drug eluting multilayered foam in accordance with the present invention may be used as a medicament to prevent or treat complications occurring by the packing, drainage, or for maintaining, opening or dilating bodily structures such as veins, arteries, uterus, urethras hollow body organs, nasal passages, sinus cavities and the like.

A further aspect of the present invention is a method to obtain a drug eluting biodegradable multilayered foam according to any of the previous claims comprising:
- providing a first polymer;
- dissolving the first polymer in a first solvent resulting in a first polymer solution;
- providing a second polymer;
- dissolving the second polymer in a second solvent resulting in a second polymer solution;
- placing the second polymer solution in contact with, preferably on top of, the first polymer solution such that the polymer solutions do not fully blend and two layers of different polymer solutions are formed;
- removing at least part of the solvent such that a multi-layered foam is obtained;
wherein at least one drug is added to at least one polymer solution before said polymer solution is placed in contact with the other polymer solution; and preferably wherein the first polymer solution is frozen before the second polymer solution is placed in contact to prevent full blending of the polymer solutions.

By preventing the polymer solutions to fully blend, the properties (*e.g.* the rate of release, *vide supra*) of the individual layers are maintained.

For the purpose of clarity and a concise description features are described herein as part of the same or separate embodiments, however, it will be appreciated that the scope of the invention may include embodiments having combinations of all or some of the features described

The invention may be illustrated by the following examples.

### Preparation of polymer

For the examples, a polyurethane-polyester copolymer comprising hydrophilic segments is selected as the polymer for the layers in the drug eluting biodegradable multilayered foam. This polyurethane-polyester copolymer is described in more detail in WO-A-2004/062704.

The first step in the preparation of the polyurethane-polyester copolymer is the synthesis of a random polyester pre-polymer, which is obtained by a ring opening polymerization of the monomers DL-lactide and ε-caprolactone with polyethylene glycol (PEG) as an initiator and tin(II) ethylhexanoate as catalyst. The resulting macrodiol is end-capped with an excess of 1,4-butanediisocyanate (BDI) to generate the isocyanate terminated prepolymer. The excess of BDI is removed by distillation under reduced pressure (1×10⁻³ mbar) at a temperature of 70°C. Subsequently, chain extension is performed by using an BDO-BDI-BDO urethane block (in the solvent 1,4-dioxane). The BDO-BDI-BDO block is prepared by reacting 1,4-butandiisocyanate with an excess of 1,4-butandiol. The excess 1,4-butanediol is removed after reaction using several washing steps (acetone/hexane) and recrystallization using chloroform. At this point, the PU block is 96.5% pure and may contain some residual side products (additional BDI-BDO group(s)) and 1,4-butanediol. Reaction with the BDO-BDI-BDO urethane block gives the final polyurethanes in a diluted solution (about 30 wt%) of 1,4-dioxane. The polyurethane can directly be diluted until the desired concentration (*e.g.* 1,9 wt%, 3,5 wt% or 4.5 wt% in 1,4-dioxane) by addition of 1,4-dioxane or they can be freeze-dried first to yield porous foams. These foams are subsequently dissolved in the solvent 1,4-dioxane.

### Manufacturing of layers for the biodegradable drug eluting foams

Formulation 1-3 are examples of drug-comprising layers with different porosity for the manufacturing biodegradable drug eluting polyurethane foams.

### Formulation 1:

The polyurethane (1.9 wt%, 4.96 g) was dissolved in anhydrous 1,4-dioxane (96.1 wt%, 250.87 g). Cyclohexane (2 wt%, 5.22 g) was added to the polymer solution and then stirred at room temperature (RT) for approximately 1 h. To this stirred solution, 360 mg of triamcinolone acetonide (a steroid model drug) was added. Stirring was continued for approximately 0.5 h (until a homogeneous transparent solution was obtained). A portion of this polymer solution was then poured into a 8-cm rectangular mold (dimensions of 8 × 2 × 1.5 cm) and frozen in a freezer at approximately -18 °C. The mold was freeze-dried overnight to yield a layer with a porosity of 97-98 %

### Formulation 2:

The polyurethane (3.5 wt%, 9.65 g) was dissolved in anhydrous 1,4-dioxane (94.5 wt%, 264.96 g). Cyclohexane (2 wt%, 5.51 g) was added to the polymer solution and then stirred at RT for approximately 1 h. To this stirred solution, 360 mg of triamcinolone acetonide was added. Stirring was continued for approximately 0.5h (until a homogeneous transparent solution was obtained). A portion of this polymer solution was then poured into a 8-cm rectangular mold (dimensions of 8 × 2 × 1.5 cm) and frozen in a freezer at approximately -18 °C. The mold was freeze-dried overnight to yield a layer with a porosity of 95-97 %.

### Formulation 3:

The polyurethane (4.5 wt%, 11.48 g) was dissolved in anhydrous 1,4-dioxane (93.5 wt%, 238.53 g). Cyclohexane (2 wt%, 5.10 g) was added to the polymer solution and then stirred at RT for approximately 1 h. To this stirred solution, 360 mg of triamcinolone acetonide was added. Stirring was continued for approximately 0.5 h (until a homogeneous transparent solution was obtained). A portion of this polymer solution was then poured into a 8-cm rectangular mold (dimensions of 8 × 2 × 1.5 cm) and frozen in a freezer at approximately -18 °C. The mold was freeze-dried overnight to yield a layer with a porosity of 93-95 %.

### Drug elution characteristics

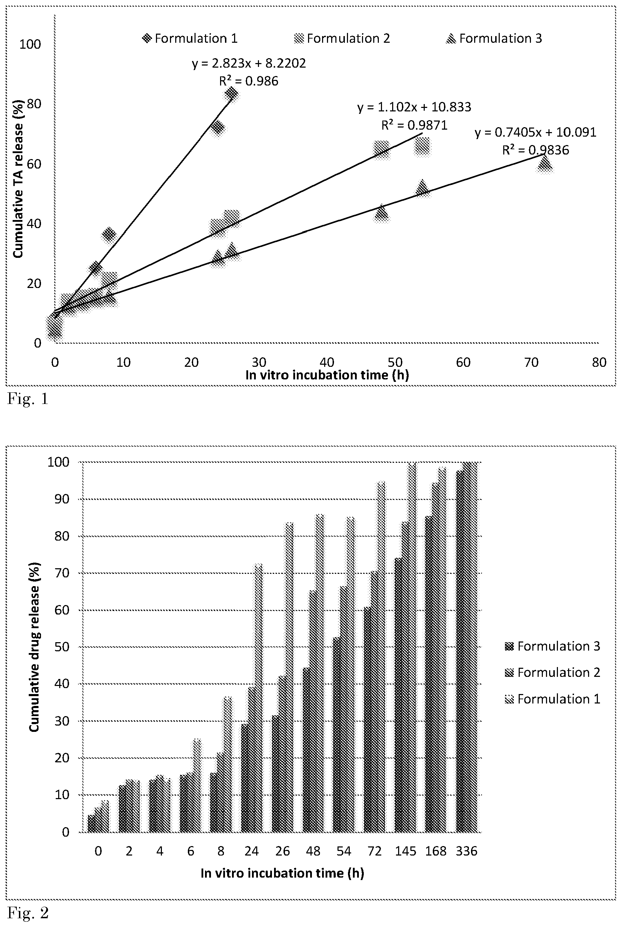

The drug release characteristics of the drug-comprising layers Formulation 1-3 are depicted in figures 1 and 2.

Figure 1 shows the tuneable period of constant drug release by varying the porosity of the foam. The illustration depicts the period of constant drug release of three different drug-comprising layers (formulation 1-3).

Figure 2 depicts the in vitro drug release profiles at different time intervals of three different drug-comprising layers (Formulation 1-3).

### Manufacturing of multilayered biodegradable drug eluting foams

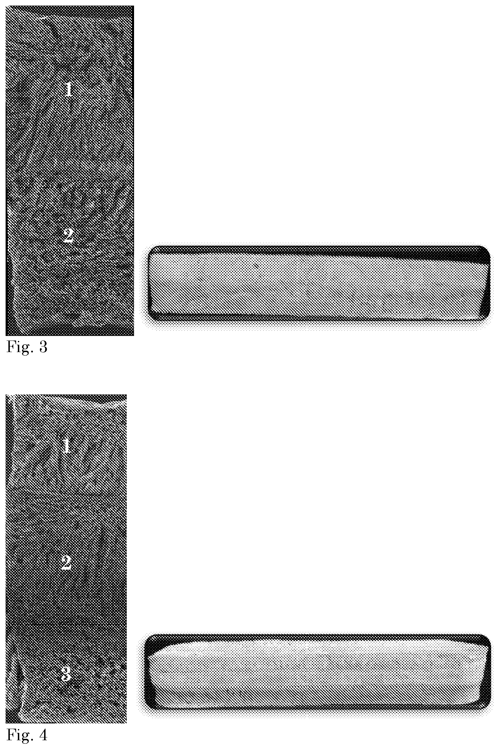

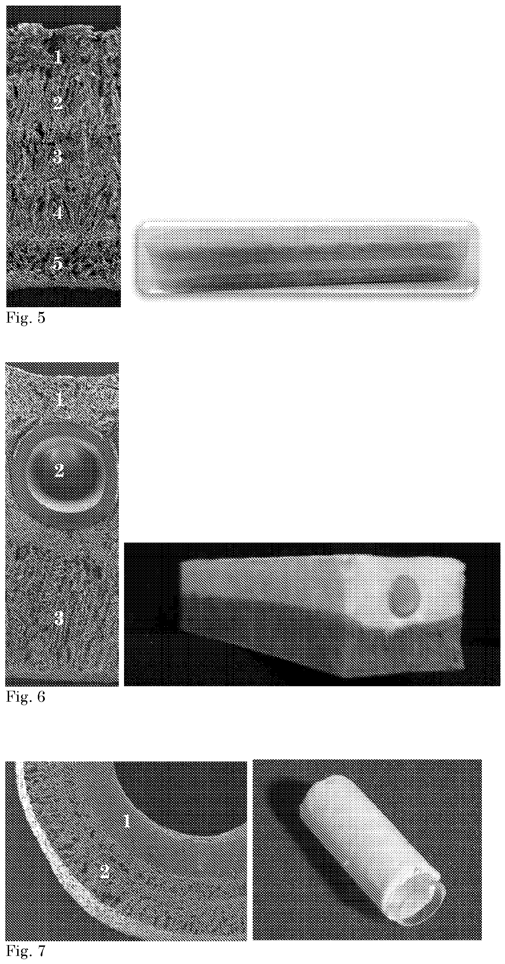

Multi-layered foams can be prepared by filling a rectangular mould with a certain amount of polymer solution (*e.g.* Formulation 3). This layer is frozen below the freezing point of the solvent. Subsequently, a second polymer solution is poured on top of the frozen layer with a different composition (*e.g.* Formulation 1). Freezing and freeze-drying results into a bilayered foam (figure 3). Foams with 3-5 layers are prepared in a similar fashion (figures 4 and 5).

## Claims

1. Multilayered drug eluting biodegradable foam comprising at least two foam layers, wherein each layer independently comprises a polymer and wherein at least one of said layers is a drug-comprising layer, which comprises at least one drug that is mixed with the polymer in said drug-comprising layer, wherein each of the at least two foam layers individually comprises a polymer that is a phase-separated polymer comprising at least one amorphous segment and at least one crystalline segment, and wherein said amorphous segment optionally comprises a hydrophilic segment.

2. Multilayered drug eluting biodegradable foam according to the previous claim, wherein at least two layers are drug-comprising foam layers which differ in one or more of the following properties:
- density;
- porosity;
- type of drugs;
- type of polymer; and
- hydrophilicity.

3. Multilayered drug eluting biodegradable foam according to any of the previous claims, wherein each layer of foam independently comprises a polymer that is selected from the group consisting of polyesters, polyethers, polyhydroxyacids, polylactones, polyetheresters, polycarbonates, polydioxanes, polyanhydrides, polyurethanes, polyester(ether)urethanes, polyurethane urea, polyamides, polyesteramides, poly-orthoesters, polyaminoacids, polyphosphonates, polyphosphazenes and combinations thereof.

4. Multilayered drug eluting biodegradable foam according to any of the previous claims, wherein said phase-separated polymer is of the formula:
-[R-Q¹[-R'-Z¹-[R"-Z²-R'-Z³]ₚ-R"-Z⁴]_{q}-R'-Q²]ₙ- (I)
wherein R is selected from one or more aliphatic polyesters, polyetheresters, polyethers, polyanhydrides and/or polycarbonates, and optionally at least one R comprises a hydrophilic segment, R' and R" are independently C₂-C₈ alkylene, optionally substituted with C₁-C₁₀ alkyl or C₁-C₁₀ alkyl groups substituted with halides or protected S, N, P or O moieties and/or comprising S, N, P or O in the alkylene chain, Z¹-Z⁴ are independently amide, urea or urethane, Q¹ and Q² are independently urea, urethane, amide, carbonate, ester or anhydride, n is an integer from 5-500, p and q are independent 0 or 1, provided that when q is 0, R is at least one amorphous aliphatic polyester, polyether, polyanhydride and/or polycarbonate segment with optionally at least one crystalline polyether, polyester, polyetherester or polyanhydride segment.

5. Multilayered drug eluting biodegradable foam according to any of the previous claims, wherein the drug-comprising layer is obtainable by
- providing the polymer;
- dissolving the polymer in a solvent resulting in a polymer solution;
- adding the drug to the polymer solution;
- blending the drug and polymer resulting in a drug-containing polymer solution;
- substantially removing the solvent, preferably by freeze-drying, from the drug-containing polymer solution to obtain the drug-comprising layer.

6. Multilayered drug eluting biodegradable foam according to any of the previous claims, wherein the drug is an anti-inflammatory agent, an hemostatic agent, an anti-allergen, an anti-cholinergic agent, an antihistamine, an anti-infective, an anti-platelet, an anti-coagulant, an anti-thrombic agent, an anti-scarring agent, an anti-proliferative agent, a chemotherapeutic agent, an anti-neoplastic agent, a pro-healing agent, decongestant, a vitamin, a hyperosmolar agent, an immunomodulator, an immunosuppressive agent, or combinations thereof, preferably wherein the drug is a steroidal anti-inflammatory agent.

7. Multilayered drug eluting biodegradable foam according to any of the previous claims, wherein said multilayered foam consists of at least three layers, typically of three- or five layers, having two outer layers and at least one internal layer, wherein said outer layers are hemostatic foams and at least one of the layers is the drug-comprising layer comprises at least one drug that is mixed with the polymers of said middle layer.

8. Method to obtain a drug eluting biodegradable multilayered foam according to any of the previous claims comprising:
- providing a first polymer;
- dissolving the first polymer in a first solvent resulting in a first polymer solution;
- providing a second polymer;
- dissolving the second polymer in a second solvent resulting in a second polymer solution;
- placing the second polymer solution in contact with, preferably on top of, the first polymer solution such that the polymer solutions do not fully blend and two layers of different polymer solutions are formed;
- removing at least part of the solvent such that a multi-layered foam is obtained;
wherein at least one drug is added to at least one polymer solution before said polymer solution is placed in contact with the other polymer solution; and preferably wherein the first polymer solution is frozen before the second polymer solution is placed in contact to prevent full blending of the polymer solutions.

9. Device, such as a tube, stent, sheet, mesh, wire or another type of foam, which surface is at least partially coated with the drug eluting foam according to any of claims 1-7.

10. Drug eluting multilayered foam in accordance with any of claims 1-7 for use as a medicament to prevent or treat complications occurring by the packing, drainage, or for maintaining, opening or dilating bodily structures such as veins, arteries, uterus, urethras hollow body organs, nasal passages, sinus cavities and the like.

## Patentansprüche

1. Mehrschichtiger, arzneimittelfreisetzender, biologisch abbaubarer Schaumstoff, umfassend wenigstens zwei Schaumstoffschichten, wobei jede Schicht unabhängig ein Polymer umfasst und wobei wenigstens eine der Schichten eine arzneimittelumfassende Schicht ist, die wenigstens ein Arzneimittel umfasst, das mit dem Polymer in der arzneimittelumfassenden Schicht gemischt ist, wobei jede der wenigstens zwei Schaumstoffschichten individuell ein Polymer umfasst, das ein phasengetrenntes Polymer ist, umfassend wenigstens ein amorphes Segment und wenigstens ein kristallines Segment, und wobei das amorphe Segment optional ein hydrophiles Segment umfasst.

2. Mehrschichtiger arzneimittelfreisetzender biologisch abbaubarer Schaumstoff nach dem vorhergehenden Anspruch, wobei wenigstens zwei Schichten arzneimittelumfassende Schaumstoffschichten sind, die sich in einer oder mehreren der folgenden Eigenschaften unterscheiden:
- Dichte;
- Porosität;
- Art der Arzneimittel;
- Art des Polymers; und
- Hydrophilie.

3. Mehrschichtiger, biologisch abbaubarer, arzneimittelfreisetzender Schaumstoff nach einem der vorhergehenden Ansprüche, wobei jede Schaumstoffschicht unabhängig ein Polymer umfasst, ausgewählt aus der Gruppe bestehend aus Polyestern, Polyethern, Polyhydroxysäuren, Polylactonen, Polyetherestern, Polycarbonaten, Polydioxanen, Polyanhydriden, Polyurethanen, Polyester(ether)urethanen, Polyurethanharnstoff, Polyamiden, Polyesteramiden, Polyorthoestern, Polyaminosäuren, Polyphosphonaten, Polyphosphazenen und Kombinationen davon.

4. Mehrschichtiger, arzneimittelfreisetzender, biologisch abbaubarer Schaumstoff nach einem der vorhergehenden Ansprüche, wobei das phasengetrennte Polymer die Formel hat:
-[R-Q¹[-R'-Z¹-[R"-Z²-R'-Z³]ₚ-R"-Z⁴]_{q}-R'-Q²]n- (I)
wobei R ausgewählt ist aus einem oder mehreren aliphatischen Polyestern, Polyetherestern, Polyethern, Polyanhydriden und/oder Polycarbonaten und optional wenigstens ein R ein hydrophiles Segment umfasst, R' und R" unabhängig C₂-C₈-Alkylen sind, optional substituiert mit C₁-C₁₀-Alkyl oder C₁-C₁₀-Alkylgruppen substituiert mit Halogeniden oder geschützten S-, N-, P- oder O-Einheiten und/oder umfassend S, N, P oder O in der Alkylenkette, Z¹-Z⁴ unabhängig Amid, Harnstoff oder Urethan sind, Q¹ und Q² unabhängig Harnstoff, Urethan, Amid, Carbonat, Ester oder Anhydrid sind, n eine ganze Zahl von 5-500 ist, p und q unabhängig 0 oder 1 sind, unter der Voraussetzung, dass, wenn q 0 ist, R wenigstens ein amorphes aliphatisches Polyester-, Polyether-, Polyanhydrid- und/oder Polycarbonatsegment mit optional wenigstens einem kristallinen Polyether-, Polyester-, Polyetherester- oder Polyanhydridsegment ist.

5. Mehrschichtiger arzneimittelfreisetzender biologisch abbaubarer Schaumstoff nach einem der vorhergehenden Ansprüche, wobei die arzneimittelumfassende Schicht erhalten werden kann durch
- Bereitstellen des Polymers;
- Auflösen des Polymers in einem Lösungsmittel, resultierend in einer Polymerlösung;
- Zusetzen des Arzneimittels zur Polymerlösung;
- Vermengen des Arzneimittels und Polymers, resultierend in einer arzneimittelhaltigen Polymerlösung;
- im Wesentlichen Entfernen des Lösungsmittels, vorzugsweise durch Gefriertrocknung, aus der arzneimittelhaltigen Polymerlösung, um die arzneimittelumfassende Schicht zu erhalten.

6. Mehrschichtiger, arzneimittelfreisetzender, biologisch abbaubarer Schaumstoff nach einem der vorhergehenden Ansprüche, wobei das Arzneimittel ein entzündungshemmendes Mittel, ein hämostatisches Mittel, ein Anti-Allergen, ein Anticholinergikum, ein Antihistaminikum, ein Anti-Infektionsmittel, ein Anti-Thrombozyt, ein Antikoagulans, ein Anti-Thrombosemittel, ein Anti-Narbenbildung Mittel, ein antiproliferatives Mittel, ein chemotherapeutisches Mittel, ein antineoplastisches Mittel, ein heilungsförderndes Mittel, ein abschwellendes Mittel, ein Vitamin, ein hyperosmolares Mittel, ein Immunmodulator, ein immunsuppressives Mittel, oder Kombinationen davon ist, vorzugsweise wobei das Arzneimittel ein steroidales entzündungshemmendes Mittel ist.

7. Mehrschichtiger, arzneimittelfreisetzender, biologisch abbaubarer Schaumstoff nach einem der vorhergehenden Ansprüche, wobei der mehrschichtige Schaumstoff aus wenigstens drei Schichten, typischerweise aus drei oder fünf Schichten, mit zwei äußeren Schichten und wenigstens einer inneren Schicht besteht, wobei die äußeren Schichten hämostatische Schaumstoffe sind und wenigstens eine der Schichten die arzneimittelumfassende Schicht ist, umfassend wenigstens ein Arzneimittel, das mit den Polymeren der mittleren Schicht gemischt ist.

8. Verfahren, um einen arzneimittelfreisetzenden, biologisch abbaubaren, mehrschichtigen Schaumstoff nach einem der vorhergehenden Ansprüche zu erhalten, umfassend:
- Bereitstellen eines ersten Polymers;
- Auflösen des ersten Polymers in einem ersten Lösungsmittel, resultierend in einer ersten Polymerlösung;
- Bereitstellen eines zweiten Polymers;
- Auflösen des zweiten Polymers in einem zweiten Lösungsmittel, resultierend in einer zweiten Polymerlösung;
- In-Kontakt-Bringen der zweiten Polymerlösung mit der ersten Polymerlösung, vorzugsweise auf die erste Polymerlösung, so dass sich die Polymerlösungen nicht vollständig vermengt und zwei Schichten unterschiedlicher Polymerlösungen gebildet werden;
- Entfernen wenigstens eines Teils des Lösungsmittels, so dass ein mehrschichtiger Schaumstoff erhalten wird;
wobei wenigstens ein Arzneimittel wenigstens einer Polymerlösung zugesetzt wird, bevor die Polymerlösung mit der anderen Polymerlösung in Kontakt gebracht wird; und vorzugsweise wobei die erste Polymerlösung eingefroren wird, bevor die zweite Polymerlösung in Kontakt gebracht wird, um ein vollständiges Vermengen der Polymerlösungen zu verhindern.

9. Vorrichtung, wie ein Röhrchen, ein Stent, eine Folie, ein Netz, ein Draht oder eine andere Art von Schaumstoff, deren Oberfläche wenigstens teilweise mit dem arzneimittelfreisetzenden Schaumstoff nach einem der Ansprüche 1 - 7 beschichtet ist.

10. Arzneimittelfreisetzender mehrschichtiger Schaumstoff nach einem der Ansprüche 1 - 7 zur Verwendung als Arzneimittel zur Verhinderung oder Behandlung von Komplikationen, auftretend bei Packung, Drainage, oder zur Aufrechterhaltung, Öffnung oder Dilatation von Körperstrukturen wie Venen, Arterien, Gebärmutter, Harnröhren, Hohlkörperorganen, Nasengängen, Nebenhöhlen und dergleichen.

## Revendications

1. Mousse biodégradable multicouche à élution de médicament comprenant au moins deux couches de mousse, dans laquelle chaque couche comprend indépendamment un polymère et dans laquelle au moins une desdites couches est une couche comprenant un médicament, qui comprend au moins un médicament qui est mélangé avec le polymère dans ladite couche comprenant un médicament, dans lequel chacune des au moins deux couches de mousse comprend individuellement un polymère qui est un polymère à phases séparées comprenant au moins un segment amorphe et au moins un segment cristallin, et dans lequel ledit segment amorphe comprend éventuellement un segment hydrophile.

2. Mousse biodégradable multicouche à élution de médicament selon la revendication précédente, dans laquelle au moins deux couches sont des couches de mousse contenant un médicament qui diffèrent par une ou plusieurs des propriétés suivantes :
- densité;
- porosité ;
- type de médicament ;
- type de polymère ; et
- hydrophilie.

3. Mousse biodégradable multicouche à élution de médicament selon l'une quelconque des revendications précédentes, dans laquelle chaque couche de mousse comprend indépendamment un polymère qui est choisi dans le groupe constitué de polyesters, polyéthers, polyhydroxyacides, polylactones, polyétheresters, polycarbonates, polydioxanes, polyanhydrides, polyuréthanes, polyester(éther)uréthanes, polyuréthane urée, polyamides, polyesteramides, poly-orthoesters, polyaminoacides, les polyphosphonates, les polyphosphazènes et leurs combinaisons.

4. Mousse biodégradable multicouche à élution de médicament selon l'une quelconque des revendications précédentes, dans laquelle ledit polymère à phases séparées répond à la formule :
-[R-Q¹[-R'-Z¹-[R"-Z²-R'-Z³]ₚ-R"-Z⁴]_{q}-R'-Q²]ₙ- (1)
dans laquelle R est choisi parmi un ou plusieurs polyesters aliphatiques, polyétheresters, polyéthers, polyanhydrides et/ou polycarbonates, et éventuellement au moins un R comprend un segment hydrophile, R' et R" sont indépendamment alkylène en C₂-C₈, éventuellement substitué par des groupes alkyle en C₁-C₁₀ ou alkyle en C₁-C₁₀ substitués par halogénures ou groupements S, N, P ou O protégés et/ou comprenant S, N, P ou O dans la chaîne alkylène, Z¹ -Z⁴ sont indépendamment amide, urée ou uréthane, Q¹ et Q² sont indépendamment urée, uréthane, amide , carbonate, ester ou anhydride, n est un nombre entier de 5 à 500, p et q sont indépendants 0 ou 1, à condition que lorsque q vaut 0, R soit au moins un segment de polyester, polyéther, polyanhydride et/ou polycarbonate aliphatique amorphe avec éventuellement au moins un segment polyéther, polyester, polyétherester ou polyanhydride cristallin.

5. Mousse biodégradable multicouche à élution de médicament selon l'une quelconque des revendications précédentes, dans laquelle la couche comprenant un médicament peut être obtenue par les étapes consistant à
- fournir le polymère ;
- dissoudre le polymère dans un solvant résultant en une solution de polymère ;
- ajouter le médicament à la solution de polymère ;
- mélanger le médicament et le polymère résultant en une solution de polymère contenant un médicament ;
- éliminer substantielle le solvant, de préférence par lyophilisation, de la solution polymère contenant le médicament pour obtenir la couche contenant le médicament.

6. Mousse biodégradable multicouche à élution de médicament selon l'une quelconque des revendications précédentes, dans laquelle le médicament est un agent anti-inflammatoire, un agent hémostatique, un anti-allergène, un agent anti-cholinergique, un antihistaminique, un anti-infectieux, un anti-plaquettaire, un anti-coagulant, un agent anti-thrombique, un agent anti-cicatrice, un agent anti-prolifératif, un agent chimiothérapeutique, un agent anti-néoplasique, un agent pro-cicatrisant, un décongestionnant, une vitamine, un agent hyperosmolaire, un immunomodulateur, un agent immunosuppresseur, ou leurs combinaisons, de préférence dans lesquelles le médicament est un agent anti-inflammatoire stéroïdien.

7. Mousse biodégradable multicouche à élution de médicament selon l'une quelconque des revendications précédentes, dans laquelle ladite mousse multicouche est constituée d'au moins trois couches, typiquement de trois ou cinq couches, ayant deux couches externes et au moins une couche interne, dans lequel lesdites couches externes sont des mousses hémostatiques et au moins l'une des couches est la couche contenant un médicament qui comprend au moins un médicament qui est mélangé avec les polymères de ladite couche médiane.

8. Procédé d'obtention d'une mousse multicouche biodégradable à élution médicamenteuse selon l'une quelconque des revendications précédentes comprenant les étapes consistant à :
- fournir un premier polymère ;
- dissoudre le premier polymère dans un premier solvant résultant en une première solution de polymère ;
- fournir un deuxième polymère ;
- dissoudre le deuxième polymère dans un deuxième solvant conduisant à une deuxième solution de polymère ;
- placer la deuxième solution de polymère en contact avec, de préférence au-dessus de, la première solution de polymère de sorte que les solutions de polymère ne soient pas complètement mélanges et deux couches de solutions polymères différentes sont formées ;
- éliminer au moins une partie du solvant de sorte qu'une mousse multicouche soit obtenue ;
dans lequel au moins un médicament est ajouté à au moins une solution de polymère avant que ladite solution de polymère ne soit mise en contact avec l'autre solution de polymère ; et de préférence dans lequel la première solution de polymère est congelée avant la deuxième la solution de polymère est mise en contact pour empêcher le mélange complet des solutions de polymère.

9. Dispositif, tel qu'un tube, un stent, une feuille, un treillis, un fil ou un autre type de mousse, dont la surface est d'au moins initialement revêtu de la mousse à élution de médicament selon l'une quelconque des revendications 1 à 7.

10. Mousse multicouche à élution de médicament selon l'une quelconque des revendications 1 à 7 destinée à être utilisée comme médicament pour prévenir ou traiter des complications survenant par le remplissage, le drainage, ou pour maintenir, ouvrir ou dilater des structures corporelles telles que les veines, les artères, l'utérus, les organes creux de l'urètre, les voies nasales, les cavités des sinus et analogues.
